# EUROPÄISCHE PATENTSCHRIFT

(11) **EP 1 794 539 B1**
(45) Veröffentlichungstag und Bekanntmachung des Hinweises auf die Patenterteilung: **05.10.2011**
(21) Anmeldenummer: 05769902.7
(22) Anmeldetag: 02.08.2005
(51) Int. Cl.: G01B 7/16, G01R 31/11

(54) **ANORDNUNG UND VERFAHREN ZUR DEGRADATIONSÜBERWACHUNG EINES BAUTEILS, MITHILFE EINES ZEITBEREICHSREFLEKTOMETERS**
ARRANGEMENT AND METHOD FOR MONITORING THE DEGRADATION OF A COMPONENT BY USING A TIME DOMAIN REFLECTOMETER
DISPOSITIF ET PROCEDE POUR SURVEILLER LA DEGRADATION D'UN COMPOSANT, A L'AIDE D'UN REFLECTOMETRE DE DOMAINE TEMPOREL

(30) Priorität: 30.09.2004 DE 102004047699
(43) Veröffentlichungstag der Anmeldung: 13.06.2007
(73) Patentinhaber: SIEMENS AKTIENGESELLSCHAFT, 80333 München (DE)
(72) Erfinder: VAN DER LAAG, Niels, 81369 München (DE)
(86) Internationale Anmeldenummer: PCT/EP2005/053762
(87) Internationale Veröffentlichungsnummer: WO 2006/034907

(56) Entgegenhaltungen:
- EP-A- 0 758 794
- DE-A1- 10 223 985
- US-A- 4 255 974
- US-B1- 6 265 880
- PATENT ABSTRACTS OF JAPAN Bd. 017, Nr. 132 (P-1504), 18. März 1993 (1993-03-18) & JP 04 310855 A (DOWA MINING CO LTD), 2. November 1992 (1992-11-02)

## Beschreibung

Die Erfindung betrifft eine Anordnung eines Bauteils und mindestens einer Kontrollvorrichtung zum Erfassen einer Degradation des Bauteils, wobei die Kontrollvorrichtung mindestens eine elektrische Leiterstruktur aufweist und das Bauteil und die Leiterstruktur der Kontrollvorrichtung derart miteinander verbunden sind, dass die Degradation des Bauteils eine Degradation der Leiterstruktur bewirkt. Daneben wird ein Verfahren zum Überprüfen einer Funktionsfähigkeit des Bauteils unter Verwendung der Anordnung angegeben.

Eine Anordnung der genannten Art ist beispielsweise aus der DE 102 23 985 A1 bekannt. Das Bauteil ist ein keramisches Hitzeschild, das in einer Brennkammer einer Gasturbine eingesetzt wird. Die Brennkammer der Gasturbine weist einen Innenraum und ein den Innenraum umgebendes Gehäuse auf. Im Innenraum der Brennkammer wird ein fossiler Brennstoff verbrannt. Bei der Verbrennung wird eine Temperatur von bis zu 1500° C erreicht. Dabei können korrosive Gase entstehen, die das Gehäuse der Brennkammer angreifen. Zum Schutz des Gehäuses vor den hohen Temperaturen und vor dem Angriff korrosiver Gase ist die Brennkammer mit einer Vielzahl keramischer Hitzeschilde ausgekleidet.

Ein Hitzeschild ist ein Bauteil aus einem Bauteilmaterial, das eine sehr gute Temperatur und Korrosionsbeständigkeit aufweist. Das Bauteilmaterial ist ein keramisches Material, beispielsweise Mullit. Aufgrund einer porösen Struktur mit einer Vielzahl von Mikrorissen zeigt Mullit ein sehr gutes Thermoschockverhalten. Eine sehr starke Temperaturschwankung, die beispielsweise beim Unterbrechen des Verbrennungsprozesses in der Brennkammer der Gasturbine auftritt, wird ausgeglichen, ohne dass das Hitzeschild zerstört wird. Allerdings kann es bei einer mechanischen Überlastung des Hitzeschildes zu einer Degradation des Hitzeschildes kommen. Es kann sich ein Riss (Makroriss) im Hitzeschild bilden. Ein derartiger Riss bildet sich dabei insbesondere an einem Rand des Hitzeschildes. Im Betrieb kann es zu einer Ausbreitung des Risses in Richtung Mitte des Hitzeschildes kommen. Der Riss wirkt sich bis zu einer bestimmten kritischen Risslänge nicht nachteilig auf die Funktionsfähigkeit des Hitzeschildes aus und kann deshalb toleriert werden. Überschreitet der Riss aber die kritische Risslänge, so ist die Funktionsfähigkeit des Hitzeschildes nicht mehr gewährleistet. Ein Austausch des Hitzeschildes ist erforderlich, um einen durch den Riss verursachten Bruch des Hitzeschildes während des Betriebs der Gasturbine zu vermeiden. Es ist also unbedingt notwendig, einen vorhandenen Riss zu erkennen und die Länge des Risses zu bestimmen.

Um einen Riss zu detektieren, der vom Rand her über die kritische Risslänge hinaus in Richtung der Mitte des Hitzeschildes fortgeschritten ist, verfügt die Kontrollvorrichtung der bekannten Anordnung über eine elektrische Leiterstruktur. Die Leiterstruktur ist derart ausgelegt, dass bei intakter Leiterstruktur ein elektrisches Signal detektiert werden kann. Das elektrische Signal ist beispielsweise eine Resonanzfrequenz der Leiterstruktur. Die Leiterstruktur ist als Schwingkreis ausgestaltet. Wenn die Leiterstruktur degradiert ist, wird kein Signal erhalten. Das Fehlen des Signals wird als Überschreiten der kritischen Risslänge interpretiert.

Es kann allerdings vorkommen, dass trotz fortgeschrittener Degradation ein Signal detektiert wird. Beispielsweise wird die Funktionsfähigkeit der Hitzeschilde der Brennkammer in einer Standphase der Gasturbine durchgeführt. Die Gasturbine wird abgeschaltet. Dabei kühlen sich die Hitzeschilde ab. Aufgrund der Abkühlung kann sich ein in einem Hitzeschild vorhandener Riss schließen, so dass sich die Enden der durch den Riss getrennten Leiterstruktur berühren. Trotz erfolgter Degradation des Hitzeschildes kann in diesem Fall ein Signal detektiert werden. Als Folge davon wird das Überschreiten der kritischen Risslänge nicht erkannt.

Aus US 6 265 880 B1 ist eine Anordnung eines Bauteils und einer Kontrollvorrichtung zum Erfassen einer Degradation des Bauteils bekannt. Die Kontrollvorrichtung basiert auf der Untersuchung eines Zeitbereichverhaltens der Leiterstruktur der Kontrollvorrichtung.

Aufgabe der vorliegenden Erfindung ist es, eine Anordnung aus Bauteil und Kontrollvorrichtung anzugeben, die eine eindeutige Aussage über die Funktionsfähigkeit des Bauteils zulässt. Darüber hinaus soll ein Verfahren angegeben werden, das eine eindeutige Aussage über die Funktionsfähigkeit des Bauteils zulässt.

Zur Lösung der Aufgabe wird eine Anordnung eines Bauteils und mindestens einer Kontrollvorrichtung zum Erfassen einer Degradation des Bauteils gemäß Anspruch 1 angegeben.

Zur Lösung der Aufgabe wird auch ein Verfahren zum Überprüfen einer Funktionsfähigkeit eines Bauteils unter Verwendung der Anordnung angegeben. Das Verfahren beinhaltet folgende Verfahrensschritte: a) Einspeisen des Eingangssignals in die Leiterstruktur, b) Auslesen des Ausgangssignals aus der Leiterstruktur und c) Auswerten des Messsignals.

Die grundlegende Idee der Erfindung besteht darin, das Zeitbereichsverhalten (Time Domain Reflectometry, TDR) der Leiterstruktur zu überprüfen. Die Leiterstruktur weist vorzugsweise mindestens zwei Verbindungsleitungen auf, die derart nahe aneinander angeordnet sind, dass eine kapazitive Kopplung zwischen den Verbindungsleitungen möglich ist. Die Leiterstruktur und das Bauelement sind derart miteinander verbunden, dass eine Degradation des Bauteils zu einer Degradation der Leiterstruktur führt. Diese Degradation führt zu einer veränderten charakteristischen Impedanz der Leiterstruktur. Diese veränderte charakteristische Impedanz kann mit Hilfe der Zeitbereichsreflektometrie erfasst werden.

Die Zeitbereichsreflektometrie ist eine Echotechnik. Es wird ein elektrisches Eingangssignal in die zu untersuchende Leiterstruktur eingespeist. Dieses Eingangssignal wird innerhalb der Leiterstruktur reflektiert. Das reflektierte Signal wird aus der Leiterstruktur ausgelesen und in ein Messsignal überführt. Bei einer intakten Leiterstruktur findet eine Reflexion an vorgegebenen Stellen der Leiterstruktur statt. Es wird ein Messsignal für die charakteristische Impedanz der Leiterstruktur erhalten. Sobald eine Störstelle in der Leiterstruktur auftritt, kommt es zur Reflexion auch an der Störstelle. Es resultiert ein Messsignal für eine veränderte Impedanz, die nicht mehr charakteristisch ist für eine intakte Leiterstruktur. Es wird ein Messsignal erhalten, mit dem Störstellen in der Leiterstruktur aufgedeckt werden können.

Durch die Degradation des Bauteils und der damit einhergehenden Degradation der Leiterstruktur wird eine Störstelle in der Leiterstruktur erzeugt. Diese Störstelle wird mit der Zeitbereichsreflektometrie aufgedeckt. Dabei ist es möglich, dass die Leiterstruktur, wie oben beschrieben, nach erfolgter Degradation wieder geheilt wurde. Auch die geheilte Leiterstruktur führt zu einer Störstelle, an der eine Reflexion stattfindet. Somit ist es möglich, unabhängig von einem möglichen Heilungsprozess der Leiterstruktur die Degradation des Bauteils aufzudecken.

Das Mittel zur Untersuchung des Zeitbereichsverhaltens der Leiterstruktur weist mindestens ein Mittel zum Erzeugen eines elektrischen Eingangssignals auf, das in die Leiterstruktur eingespeist wird, und mindestens ein Mittel zum Erzeugen eines Messsignals aus einem durch Reflexion des Eingangssignals in der Leiterstruktur hervorgerufenen elektrischen Ausgangssignals.

Um das eingespeiste Eingangssignal und das auszulesende Ausgangssignal voneinander unterscheiden zu können, weist das Mittel zur Untersuchung des Zeitbereichsverhaltens der Leiterstruktur mindestens ein Mittel zum Erzeugen einer zeitlichen Verzögerung des Eingangssignals und/oder des Messsignals auf.

Das Mittel zum Erzeugen des Eingangssignals, des Messsignals und der zeitlichen Verzögerung weist mindestens ein Oberflächenwellenbauelement auf. Das Oberflächenwellenbauelement basiert auf der Wandlung eines elektrischen Signals, mit dem die Elektroden auf dem Oberflächenabschnitt des piezoelektrischen Trägerkörpers (Substrat) angesteuert werden, in eine mechanische Oberflächenwelle (Surface Acoustic Wave, SAW) am Oberflächenabschnitt. Dazu weist das Oberflächenwellenbauelement mindestens einen piezoelektrischen Trägerkörper und mindestens zwei an einem Oberflächenabschnitt des Trägerkörpers angeordnete Elektroden zum Erzeugen einer mechanischen Oberflächenwelle am Oberflächenabschnitt des Trägerkörpers auf. Die Elektroden und der Trägerkörper bilden zusammen eine Oberflächenwellenstruktur des Oberflächenwellenbauelements. Der Trägerkörper ist vorzugsweise aus einem einkristallinen piezoelektrischen Material. Vorzugsweise bilden die Elektroden eine Interdigitalstruktur. Die Interdigitalstruktur ist ein Bestandteil eines Interdigitalwandlers.

In einer besonderen Ausgestaltung weist das Oberflächenwellenbauelement am Oberflächenabschnitt des Trägerkörpers mindestens einen weiteren Interdigitalwandler zum Erzeugen des Eingangssignals und/oder zum Erzeugen des Messsignals auf. Vorteilhaft ist dabei der weitere Interdigitalwandler derart mit einer Antenne zum Empfangen eines elektromagnetischen Abfragesignals und/oder zum Senden eines elektromagnetischen Messsignals verbunden, dass das Abfragesignal durch den weiteren Interdigitalwandler in eine Abfrageoberflächenwelle und/oder eine Messoberflächenwelle durch den weiteren Interdigitalwandler in das Messsignal umgewandelt werden können. Aus dem empfangenen Abfragesignal wird die Abfrageoberflächenwelle erzeugt, die in das Eingangssignal umgewandelt wird. Zum Auswerten des Ausgangssignals wird das Ausgangssignal in eine Messoberflächenwelle umgewandelt. Die Messoberflächenwelle wird in das Messsignal umgewandet. Das Messsignal wird über die Antenne ausgesandt und von einer passenden Detektoreinheit erfasst. Dies bedeutet, dass das Erzeugen der Abfrageoberflächenwelle berührungslos erfolgt. Vorteilhaft erfolgt die Detektion des Messsignals ebenfalls berührungslos. Die Möglichkeit, die Funktionsfähigkeit des Bauteils berührungslos zu überprüfen, ist insbesondere für schwer zugängliche Bauteile von Vorteil. Denkbar ist dabei, dass ein und derselbe Interdigitalwandler zum Erzeugen der Abfrageoberflächenwelle und zum Umwandeln der Messoberflächenwelle in das Messsignal verwendet wird. Es können auch verschiedene Antennen und/oder Interdigitalwandler zum Erzeugen der Abfrageoberflächenwelle und zum Erzeugen des Messsignals verwendet werden.

Die Kombination des Sendens des elektromagnetischen Abfragesignals mit der Verzögerung durch das Oberflächenwellenbauelements ist besonders günstig, da eine Ausbreitungsgeschwindigkeit der Oberflächenwelle auf dem Oberflächenabschnitt des Trägerkörpers um etwa den Faktor 10⁵

Langsamer erfolgt, als die Ausbreitungsgeschwindigkeit elektromagnetischer Wellen. Dadurch wird eine effiziente Verzögerung und damit eine zeitliche Trennung des Ausgangssignals und nach Reflexion in der Leiterstruktur erhaltenen Messsignals erzielt.

Wichtig ist, das die Leiterstruktur und das Bauteil derart ausgestalten sind, dass die (kritische) Degradation des Bauteils zu einer Degradation der Leiterstruktur und damit zu einem detektierbaren Messsignal führt. Dazu können das Bauteilmaterial des Bauteils und das Leitermaterial der Leiterstruktur aus völlig verschiedenen Materialien bzw. Materialklassen mit unterschiedlichen mechanischen Eigenschaften bestehen. Beispielsweise besteht das Bauteil aus einem Metall. Aufgrund einer Duktilität des Bauteils kann es zu einer Degradation des Bauteils in Form einer Verbiegung kommen. Damit die Verbiegung mit Hilfe der Leiterstruktur bestimmt werden kann, wird beispielsweise die Leiterstruktur elektrisch isolierend auf der Oberfläche des Bauteils aufgebracht. Als elektrischer Isolator fungiert beispielsweise eine Keramik. Wenn die Leiterstruktur aus einem spröden Leitermaterial gebildet ist, führt die Verbiegung des Bauteils zu einer Degradation in Form eines Risses in der Leiterstruktur. Der Riss führt zur elektrischen Isolierung der Elektroden des Oberflächenwellenbauelements voneinander. Wie oben geschildert, führt der Riss der Leiterstruktur zu einem detektierbaren Messsignal. Die Ursache des Risses, also die Verbiegung des Bauteils kann nachgewiesen werden.

In einer besonderen Ausgestaltung weisen ein Bauteilmaterial des Bauteils und das Leitermaterial der Leiterstruktur eine im Wesentlichen gleiche mechanische Eigenschaft auf. Diese mechanische Eigenschaft ist insbesondere aus der Gruppe Temperaturausdehnungsverhalten und Bruchzähigkeit ausgewählt. Bei einem Bauteil in Form eines Hitzeschildes tritt zwischen einer Betriebsphase und einer Standphase der Gasturbine ein sehr großer Temperaturunterschied auf. Im Betrieb wird beispielsweise im Innenraum der Brennkammer eine Temperatur von bis zu 1500° C erreicht. Durch das im Wesentlichen gleiche Temperaturausdehnungsverhalten ist gewährleistet, dass der Kontakt zwischen der Leiterstruktur und dem Bauteil auch während eines Wechsels zwischen Betriebsphase und Standphase bestehen bleibt. Besonders vorteilhaft ist es, wenn die Bruchzähigkeit des Bauteilmaterials und die Bruchzähigkeit des Leitermaterials im Wesentlichen gleich sind. Dadurch wird erreicht, dass sich ein Bruch oder ein Riss im Bauteil in die Leiterstruktur fortsetzen kann. Bei einem Bauteil aus einem keramischen Bauteilmaterial ist dazu die Leiterstruktur vorteilhaft aus einem keramischen Leitermaterial.

Als Trägermaterial des Trägerkörpers des Oberflächenwellenbauelements ist ein beliebiges, piezoelektrisches Material wie Quarz, Lithiumniobat oder Galliumphosphat (GaPO₄) denkbar. Insbesondere weist das Trägermaterial mindestens ein Seltenerdoxid auf. Das Seltenerdoxid kann insbesondere ein Oxid mit mehreren Seltenerdmetallen und/oder Metallen der Haupt- und Nebengruppen des Periodensystems sein. Das Oxid des Seltenerdmatalls ist insbesondere ein Langasit. Zur Langasit-Gruppe gehören neben Langasit (La₃Ga₅SiO₁₄), Neodym-Langasit (Nd₃Ga₅SiO₁₄) oder Praseodym-Langasit (Pr₃Ga₅SiO₁₄) auch die Langanat-Gruppe (z.B. La₃Ga_{5,5}Nb_{0,5}O₁₄) und die Langatat-Gruppe (z.B. La₃Ga_{5,5}Ta_{0,5}O₁₄). Das Langasit kann also auch ein Langanat oder ein Langatat sein. Oberflächenwellenbauelemente mit diesen Materialien sind besonders für Anwendungen von über 600 °C geeignet.

Die Kontrollvorrichtung ist zum Erfassen der Degradation eines beliebigen Bauteils einsetzbar. Mit der Ausgestaltung des berührungslosen Erzeugens der Abfrageoberflächenwelle und des berührungslosen Detektieren des Messsignals eignet sich die Kontrollvorrichtung insbesondere zur Funktionskotrolle eines schwer zugänglichen Bauteils.

Die Kontrollvorrichtung wird insbesondere zur Überprüfung der Funktionsfähigkeit eines Hitzeschildes einer Brennkammer einer Gasturbine verwendet. Dazu werden vorzugsweise die oben erwähnten Trägermaterialien für den oder die Trägerkörper der Oberflächenwellenbauelemente der Hitzeschilde verwendet. In einer besonderen Ausgestaltung ist die Leiterstruktur an einer Oberfläche des Hitzeschildes angeordnet, die einem Innenraum der Brennkammer abgekehrt ist. Die Hitzeschilde der Gasturbine können, obwohl sie schwer zugänglich sind, jederzeit und insbesondere in der Betriebsphase der Gasturbine auf ihre Funktionsfähigkeit hin überprüft werden. Dazu ist jeweils die Leiterstruktur derart auf einer Oberfläche eines Hitzeschildes angeordnet, dass das Überschreiten der kritischen Risslänge automatisch zu einer Degradation der Leiterstruktur führt.

Neben der Anwendung zur Funktionskontrolle sind viele weitere Anwendungen denkbar. So kann beispielsweise eine Funktionsüberprüfung tragender Teile von Gebäuden auf elegante Weise überprüft werden. Dies kann in regelmäßigen Abständen im Zusammenhang mir Wartungsarbeiten erfolgen. Denkbar ist auch, dass die Funktionskontrolle nach singulären Ereignissen durchgeführt wird. Beispielsweise werden tragende Teile nach einem Erdbeben auf ihre Funktion hin überprüft. Vorhandene Risse der tragenden Teile können aufgedeckt werden.

Mit der Erfindung ergeben sich zusammenfassend folgende wesentliche Vorteile:
- Die Erfindung führt zu einem eindeutigen Nachweis der Degradation eines Bauteils.
- Der Nachweis der Degradation kann berührungslos erfolgen.
- Aufgrund der Empfindlichkeit der Zeitbereichsreflektometrie auf Störstellen kann auch eine temporäre Degradation der Leiterstruktur aufgedeckt werden. Somit ist ein sicherer Nachweis der Degradation des Bauteils möglich.

Anhand der Figuren wird die Erfindung im Folgenden näher beschrieben. Die Figuren sind schematisch und stellen keine maßstabsgetreuen Abbildungen dar.
- Figur 1: zeigt eine Anordnung eines Bauteils und einer Kon- trollvorrichtung in perspektivischer Darstellung.
- Figur 2: zeigt eine Anordnung eines Bauteils und einer Kon- trollvorrichtung inklusive Antenne zum Senden eines Abfragesignals und Empfangen eines Messsignals.
- Figur 3: zeigt einen Ausschnitt einer Anordnung eines Bauteils und der Leiterstruktur der Kontrollvorrichtung, wobei sich die Degradation des Bauteils als Degradation der Leiterstruktur fortsetzt.
- Figur 4: zeigt ein Oberflächenwellenbauelement der Kontroll- vorrichtung.
- Figur 5A: zeigt ein Messsignal der Kontrollvorrichtung bei in- takter Leiterstruktur.
- Figur 5B: ein Messsignal der Kontrollvorrichtung bei degra- dierter Leiterstruktur.

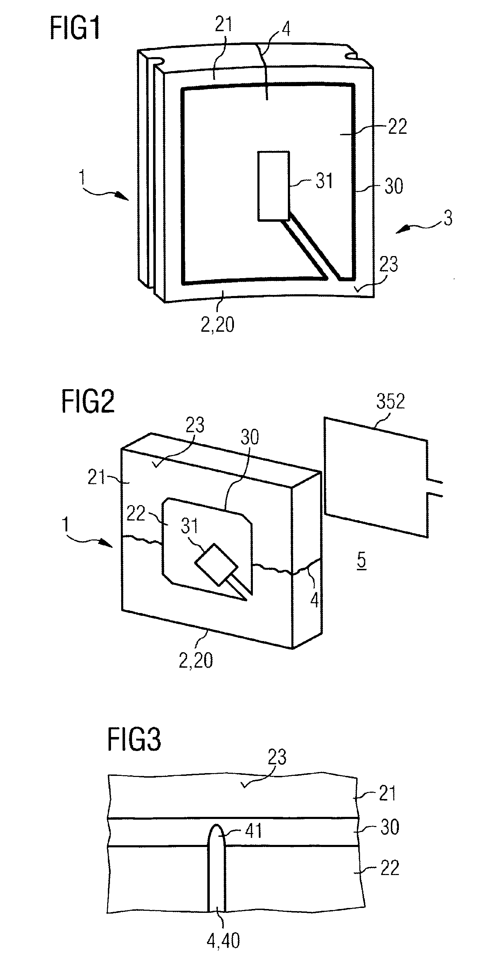

Die Anordnung 1 besteht aus einem Bauteil 2 in Form eines Hitzeschildes 20 der Brennkammer einer Gasturbine und einer Kontrollvorrichtung 3 zum Erfassen einer Degradation 4 des Hitzeschildes 20 (Figur 1). Das Hitzeschild 20 weist als Bauteilmaterial eine Keramik auf. Die Keramik ist Mullit.

Die Kontrollvorrichtung 3 weist eine auf der Oberfläche 23 des Hitzeschildes 20 aufgebrachte elektrische Leiterstruktur 30 auf. Die Leiterstruktur 30 weist zwei aneinander angeordnete elektrische Verbindungsleitungen 301 und 302 auf. Die Verbindungsleitungen 301 und 302 sind so nahe aneinander angeordnet, dass eine kapazitive Kopplung zwischen der Verbindungsleitungen 301 und 302 möglich ist. Das Leitermaterial der Leiterstruktur 30 ist ein bis zu einer Temperatur von 800° C beständiger, elektrisch leitfähiger keramischer Leiter. Das Leitermaterial und das Bauteilmaterial sind spröde. Sie zeigen eine im Wesentlichen gleiche Bruchzähigkeit auf.

Die Oberfläche 23 des Hitzeschildes 20, auf der die Leiterstruktur 30 angeordnet ist, ist dem Innenraum 5 der Brennkammer der Gasturbine abgekehrt (Figur 2). Die Leiterstruktur 30 befindet sich dabei an einem Rand 21 des Hitzeschildes 20.

Die Leiterstruktur 30 ist auf dem Oberflächenabschnitt 23 des Hitzeschildes 20 derart aufgebracht, dass sich jeder Riss 4, 40 im Hitzeschild 20, der sich vom Rand 21 des Hitzeschildes 20 in Richtung Mitte 22 des Hitzeschildes 20 ausbreitet und der eine bestimmte kritische Risslänge überschreitet, in der Leiterstruktur 30 fortsetzt (Figur 3). Sobald die Länge des Risses 40 die kritische Risslänge überschreitet, kommt es zu einer Degradation 41 (Rissbildung bzw. Rissausbreitung) der Leiterstruktur 30.

Die Kontrollvorrichtung 3 weist ein Oberflächenwellenbauelement 31 auf, das in der Mitte 22 der Oberfläche 23 des Hitzeschildes 20 aufgebracht ist (Figuren 1, 2 und 3). Das Oberflächenwellenbauelement 31 weist einen piezoelektrischen Trägerkörper 32 aus Langasit mit der Summenformel La₃Ga₅SiO₁₄ auf. Der Trägerkörper 32 verfügt über einen Interdigitalwandler 34 und einen weiteren Interdigitalwandler 35. Jeder der Interdigitalwandler 34 und 35 verfügt über eine am Oberflächenabschnitt 33 des Trägerkörpers 32 angeordnete Interdigitalstruktur. Die Interdigitalstruktur ist eine Kondensatorstruktur mit fingerartig ineinander greifenden Elektroden. Die Elektroden 341 und 342 des Interdigitalwandlers 34 sind dabei über eine Verstärkereinheit 36 mit der Leiterstruktur 30 der Kontrollvorrichtung 3 derart verbunden, so dass eine an dem Oberflächenwellenabschnitt 33 erzeugte Abfrageoberflächenwelle als Eingangssignal in die Verbindungsleitungen 301 und 302 der Leiterstruktur 30 eingespeist werden.

Zur Überprüfung der Funktionsfähigkeit des Hitzeschildes 20 wird auf dem Oberflächenabschnitt 33 des Trägerkörpers 32 des Oberflächenwellenbauelements 31 eine mechanische Abfrageoberflächenwelle 37 erzeugt. Dazu wird der weitere Interdigitalwandler 35 verwendet, der als Generator der Abfrageoberflächenwelle 37 fungiert. Der Interdigitalwandler 35 verfügt über eine Antenne 351. Über diese Antenne 351 wird ein hochfrequentes, elektromagnetisches Abfragsignal eingekoppelt, das von einer Sendeantenne 352 ausgesandt wird (Figur 2). Das Abfragesignal ist ein elektromagnetischer Abfragepuls. Der weitere Interdigitalwandler 35 transformiert das hochfrequente Abfragesignal in die mechanische Abfrageoberflächenwelle 37. Die Abfrageoberflächenwelle 37 breitet sich entlang des Oberflächenabschnitts 33 des Trägerkörpers 32 aus.

Am Interdigitalwandler 34 wird die Abfrageoberflächenwelle 39 in das Eingangssignal umgewandelt. Das Eingangssignal ist ein hochfrequenter elektrischer Puls. In der Leiterstruktur 30 kommt es zur Reflexion des Eingangssignals. Das reflektierte Eingangssignal wird als Ausgangssignal über die Verstärkereinheit 36 erfasst und über den Interdigitalwandler 34 in die Messoberflächenwelle 371 überführt. Am weiteren Interdigitalwandler 35 erfolgt die Umwandlung der Messoberflächenwelle 371 in ein Messsignal, das von der Antenne 351 ausgesendet wird.

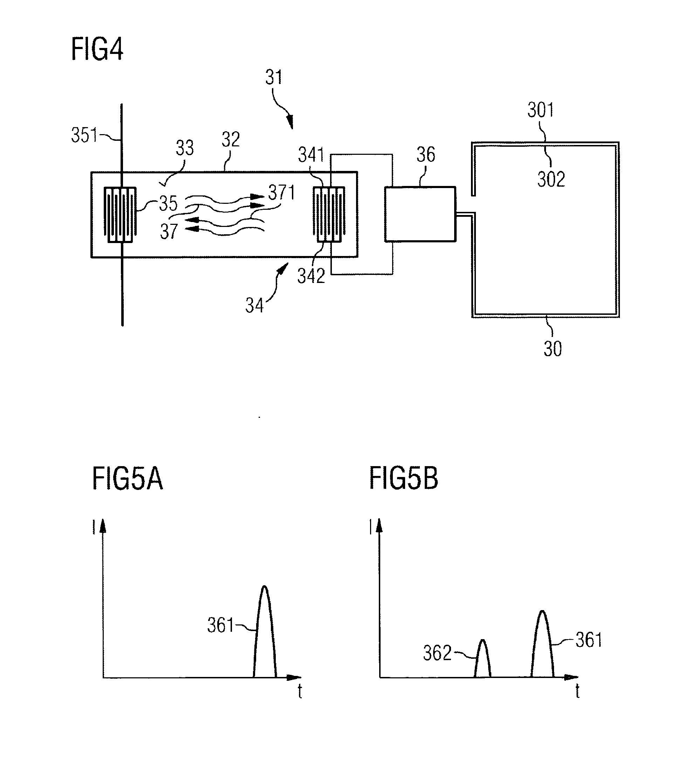

Bei intakter Leiterstruktur 30 wird ein Messsignal mit einem charakteristischen Intensitätsmaximum 361 erhalten, das auf der charakteristischen Impedanz der Leiterstruktur 30 bzw. der Verbindungsleitungen 301 und 302 der Leiterstruktur beruht (Figur 5A).

Ist die Leiterstruktur 30 degradiert, wird ein Messsignal erhalten, das neben dem charakteristischen Intensitätsmaximum 361 ein weiteres Intensitätsmaximum 362 aufweist (Figur 5B). Das weitere Intensitätsmaximum 362 beruht auf der durch die Degradation der Leiterstruktur 30 resultierenden Störstelle. Dabei ist die Leiterstruktur aus dem keramischen Leiterwerkstoff nach der durch die Degradation des Hitzeschildes hervorgerufenen Degradation wieder zusammengewachsen.

Das Auftreten des zusätzlichen Intensitätsmaximums 362 gibt den Hinweis darauf, dass die kritische Risslänge im Hitzeschild 20 überschritten ist. Das Hitzeschild 20 ist auszutauschen.

Weitere Ausführungsbeispiele ergeben sich daraus, dass mehrere Oberflächenwellenbauelemente 31, mehrere Leiterstrukturen 30, mehrere Sende- und Empfangsantennen und/oder unterschiedliche Trägermaterialien für die jeweiligen piezoelektrischen Trägerkörper 32 verwendet werden.

## Patentansprüche

1. Anordnung eines Bauteils (2, 20) und mindestens einer Kontrollvorrichtung (3) zum Erfassen einer Degradation (4, 40) des Bauteils (2), wobei
- die Kontrollvorrichtung (3) mindestens eine elektrische Leiterstruktur (30) aufweist,
- das Bauteil (2, 20) und die Leiterstruktur (30) der Kontrollvorrichtung (3) derart miteinander verbunden sind, dass die Degradation (40) des Bauteils (2) eine Degradation (41) der Leiterstruktur (30) bewirkt,
- die Kontrollvorrichtung mindestens ein Mittel zur Untersuchung eines Zeitbereichsverhaltens der Leiterstruktur (30) aufweist,
- das Mittel zur Untersuchung des Zeitbereichsverhaltens der Leiterstruktur (30) mindestens ein Mittel zum Erzeugen eines elektrischen Eingangssignals aufweist, das in die Leiterstruktur (30) eingespeist wird, zum Erzeugen eines Messsignals aus einem durch Reflexion des Eingangssignals in der Leiterstruktur (30) hervorgerufenen elektrischen Ausgangssignals, und zum Erzeugen einer zeitlichen Verzögerung des Eingangssignals und/oder des Messsignals aufweist,
**dadurch gekennzeichnet, dass**
- das Mittel zum Erzeugen des Eingangssignals, des Messsignals und der zeitlichen Verzögerung mindestens ein Oberflächenwellenbauelement (31) aufweist.

2. Anordnung nach Anspruch 1, wobei das Oberflächenwellenbauelement (30)
- mindestens einen piezoelektrischen Trägerkörper (32) und
- mindestens zwei an einem Oberflächenabschnitt (33) des Trägerkörpers (32) angeordnete Elektroden (341, 342) zum Erzeugen einer mechanischen Oberflächenwelle am Oberflächenabschnitt (33) des Trägerkörpers (32) aufweist.

3. Anordnung nach Anspruch 2, wobei die Elektroden (341, 342) eine Interdigitalstruktur bilden.

4. Anordnung nach Anspruch 3, wobei die Interdigitalstruktur Bestandteil eines Interdigitalwandlers (34) ist.

5. Anordnung nach Anspruch 4, wobei das Oberflächenwellenbauelement (31) am Oberflächenabschnitt (33) des Trägerkörpers (32) mindestens einen weiteren Interdigitalwandler (35) zum Erzeugen des Eingangssignals und/oder zum Erzeugen des Messsignals aufweist.

6. Anordnung nach Anspruch 5, wobei der weitere Interdigitalwandler (35) zum Erzeugen einer Abfrageoberflächenwelle (37) derart mit einer Antenne (351) zum Empfangen eines elektromagnetischen Abfragesignals und/oder zum Senden des elektromagnetischen Messsignals verbunden ist, dass das Abfragesignal durch den weiteren Interdigitalwandler (35) in die Abfrageoberflächenwelle (37) und/oder die die eine Messoberflächenwelle (37) durch den weiteren Interdigitalwandler (35) in das Messsignal umgewandelt werden können.

7. Anordnung nach einem der Ansprüche 1 bis 6, wobei ein Bauteilmaterial des Bauteils (2, 20) und ein Leitermaterial der Leiterstruktur (30) eine im Wesentlichen gleiche mechanische Eigenschaft aufweisen.

8. Anordnung nach einem der Ansprüche 2 bis 7, wobei der Trägerkörper (32) des Oberflächenwellenbauelements (31) ein Trägermaterial mit mindestens einem Seltenerdoxid aufweist.

9. Anordnung nach Anspruch 8, wobei das Seltenerdoxid ein Langasit ist.

10. Anordnung nach Anspruch 9, wobei das Langasit ein Langanat oder ein Langatat ist.

11. Anordnung nach einem der Ansprüche 1 bis 10, wobei das Bauteil (2) ein Hitzeschild (20) einer Brennkammer ist.

12. Anordnung nach Anspruch 11, wobei die Leiterstruktur (30) an einer Oberfläche (23) des Hitzeschildes (20) angeordnet ist, die einem Innenraum (5) der Brennkammer abgekehrt ist.

13. Verfahren zum Überprüfen einer Funktionsfähigkeit eines Bauteils (2, 20) unter Verwendung einer Anordnung nach einem Ansprüche 1 bis 12 mit folgenden Verfahrensschritten:
a) Einspeisen der Eingangssignals in die Leiterstruktur,
b) Auslesen des Ausgangssignals aus der Leiterstruktur und
c) Auswerten des Ausgangssignals.

14. Verfahren nach Anspruch 13, wobei zum Einspeisen des Eingangssignals eine Abfrageoberflächenwelle (37) erzeugt wird, die in das Eingangssignal umgewandelt wird.

15. Verfahren nach Anspruch 13 oder 14, wobei zum Auswerten des Ausgangssignals das Ausgangssignal in eine Messoberflächenwelle umgewandelt wird.

16. Verfahren nach Anspruch 14 oder 15, wobei als elektrisches Abfragesignal und/oder als elektrisches Messsignal ein elektromagnetisches Hochfrequenzsignal verwendet wird.

## Claims

1. Arrangement of a component (2, 20) and at least one control facility (3) for detecting a degradation (4, 40) of the component (2), whereby
- the control facility (3) has at least one electrical conductor structure (30),
- the component (2, 20) and the conductor structure (30) of the control facility (3) are connected to each other such that the degradation (40) of the component (2) brings about a degradation (41) of the conductor structure (30),
- the control facility has at least one means for investigating a time range characteristic of the conductor structure (30),
- the means for investigating a time range characteristic of the conductor structure (30) has at least one means for creating an electrical input signal which is injected into the conductor structure (30), for creating a measurement signal from an electrical output signal caused by reflection of the input signal in the conductor structure (30) and
for creating a time delay of the input signal and/or of the measurement signal,
**characterised in that**
- the means for generating the input signal, the measurement signal and the time delay have at least one surface acoustic wave component (31).

2. Arrangement according to claim 1, whereby the surface acoustic wave component (30)
- has at least one piezoelectric support body (32) and at least two electrodes (341, 342) arranged on a surface section (33) of the support body (32) for creating a mechanical surface acoustic wave on the surface section (33) of the support body (32).

3. Arrangement according to claim 2, whereby the electrodes (341, 342) form an interdigital structure.

4. Arrangement according to claim 3, whereby the interdigital structure is an element of an interdigital converter (34).

5. Arrangement according to claim 4, whereby the surface acoustic wave component (31) has at least one further interdigital converter (35) on the surface section (33) of the carrier body (32) for creating the input signal and/or for creating the measurement signal.

6. Arrangement according to claim 5, whereby the further interdigital converter (35) for creating a surface interrogation wave (37) is connected to an antenna (351) for receiving an electromagnetic interrogation signal and/or for sending the electromagnetic measurement signal such that the interrogation signal can be converted by the further interdigital converter (35) into the surface interrogation wave (37) and/or the surface measurement wave (37) can be converted by the further interdigital converter (35) into the measurement signal.

7. Arrangement according to one of claims 1 to 6, whereby a component material of the component (2, 20) and a conductor material of the conductor structure (30) have a mechanical property that is essentially the same.

8. Arrangement according to one of claims 2 to 7, whereby the support body (32) of the surface acoustic wave element (31) has a support material with at least one rare earth oxide.

9. Arrangement according to claim 8, whereby the rare earth oxide is a langasite.

10. Arrangement according to claim 9, whereby the langasite is a langanate or a langatate.

11. Arrangement according to one of claims 1 to 10, whereby the component (2) is a heat (20) shield of a combustion chamber.

12. Arrangement according to claim 11, whereby the conductor structure (30) is arranged on a surface (23) of the heat shield (20) which faces away from an interior space (5) of the combustion chamber.

13. A method for checking a functional capability of a component (2, 20) using an arrangement according to one of claims 1 to 12 with the following method steps:
a) Injecting the input signal into the conductor structure,
b) Reading out the output signal from the conductor structure and
c) Evaluating the output signal.

14. Method according to claim 13, whereby, for injecting the input signal, a surface acoustic wave (37) is created, which is converted into the input signal.

15. Method according to claim 13 or 14, whereby, for evaluating the output signal, the output signal is converted into a surface measurement wave.

16. Method according to claim 14 or 15, whereby a highfrequency electromagnetic signal is used as the electrical interrogation signal and/or as the electrical measurement signal.

## Revendications

1. Disposition d'un composant (2, 20) et d'au moins un dispositif de contrôle (3) pour détecter une dégradation (4, 40) du composant (2), dans laquelle
- le dispositif de contrôle (3) présente au moins une structure de conducteur électrique (30),
- le composant (2, 20) et la structure de conducteur (30) du dispositif de contrôle (3) sont reliés ensemble de sorte que la dégradation (40) du composant (2) provoque une dégradation (41) de la structure de conducteur (30),
- le dispositif de contrôle comprend au moins un moyen pour étudier un comportement dans le domaine temporel de la structure de conducteur (30),
- le moyen pour étudier le comportement dans le domaine temporel de la structure de conducteur (30) présente au moins un moyen pour produire un signal d'entrée électrique, qui est injecté dans la structure de conducteur (30), pour produire un signal de mesure à partir d'un signal de sortie électrique provoqué par la réflexion du signal d'entrée dans la structure de conducteur (30), et pour produire un retard temporel du signal d'entrée et/ou du signal de mesure,
**caractérisée en ce que**
- le moyen pour produire le signal d'entrée, le signal de mesure et le retard temporel présente au moins un composant à ondes de surface (31).

2. Disposition selon la revendication 1, dans laquelle le composant à ondes de surface (30) présente :
- au moins un substrat piézo-électrique (32) et
- au moins deux électrodes (341, 342) disposées sur une section de surface (33) du substrat (32) pour produire une onde mécanique de surface sur la section de surface (33) du substrat (32).

3. Disposition selon la revendication 2, dans laquelle les électrodes (341, 342) forment une structure interdigitée.

4. Disposition selon la revendication 3, dans laquelle la structure interdigitée est un composant d'un transducteur interdigité (34).

5. Disposition selon la revendication 4, dans laquelle le composant à ondes de surface (31) sur la section de surface (33) du substrat (32) présente au moins un transducteur interdigité supplémentaire (35) pour produire le signal d'entrée et/ou pour produire le signal de mesure.

6. Disposition selon la revendication 5, dans laquelle le transducteur interdigité supplémentaire (35) pour produire une onde de surface d'interrogation (37) est relié à une antenne (351) pour recevoir un signal d'interrogation électromagnétique et/ou pour émettre le signal de mesure électromagnétique, de sorte que le signal d'interrogation peut être transformé par le transducteur interdigité supplémentaire (35) en l'onde de surface d'interrogation (37) et/ou que ladite une onde de surface de mesure (37) peut être transformée en le signal de mesure par le transducteur interdigité supplémentaire (35).

7. Disposition selon l'une des revendications 1 à 6, dans laquelle un matériau de composant du composant (2, 20) et un matériau conducteur de la structure de conducteur (30) présentent une propriété mécanique essentiellement identique.

8. Disposition selon l'une des revendications 2 à 7, dans laquelle le substrat (32) du composant à ondes de surface (31) présente un matériau de substrat ayant au moins un oxyde de terre rare.

9. Disposition selon la revendication 8, dans laquelle l'oxyde de terre rare est une langasite.

10. Disposition selon la revendication 9, dans laquelle la langasite est un langanate ou un langatate.

11. Disposition selon l'une des revendications 1 à 10, dans laquelle le composant (2) est un bouclier thermique (20) d'une chambre de combustion.

12. Disposition selon la revendication 11, dans laquelle la structure de conducteur (30) est disposée sur une surface (23) du bouclier thermique (20) qui fait dos à un espace intérieur (5) de la chambre de combustion.

13. Procédé pour contrôler le bon fonctionnement d'un composant (2, 20) en employant une disposition selon l'une des revendications 1 à 12 avec les étapes de procédé suivantes :
a) injection du signal d'entrée dans la structure de conducteur,
b) lecture du signal de sortie de la structure de conducteur et
c) évaluation du signal de sortie.

14. Procédé selon la revendication 13, dans lequel pour injecter le signal d'entrée, on produit une onde de surface d'interrogation (37) qui est transformée en le signal d'entrée.

15. Procédé selon la revendication 13 ou 14, dans lequel pour évaluer le signal de sortie, le signal de sortie est transformé en une onde de surface de mesure.

16. Procédé selon la revendication 14 ou 15, dans lequel un signal électromagnétique de haute fréquence est employé en tant que signal d'interrogation et/ou signal électrique de mesure.
